# EUROPEAN PATENT APPLICATION

(11) **EP 0 568 946 A1**
(43) Date of publication of application: **10.11.1993**
(21) Application number: 93107105.4
(22) Date of filing: 01.05.1993
(51) Int. Cl.: C07D 493/08, C07H 19/01, C12P 17/18, A61K 31/34

(54) **Compound TAN-1607A, its derivatives, their production, and use thereof**

(30) Priority: 08.05.1992 JP 115874/92
(71) Applicant: TAKEDA CHEMICAL INDUSTRIES, LTD., Chuo-ku, Osaka 541 (JP)
(72) Inventor: Kitano, Kazuaki, Sakai, Osaka 590-01 (JP); Tsubotani, Shigetoshi, Kawanishi, Hyogo 666-01 (JP); Tozawa, Ryuichi, Ibaraki, Osaka 567 (JP); Harada, Setsuo, Kawanishi, Hyogo 666-01 (JP)
(74) Representative: von Kreisler, Alek, Dipl.-Chem.

(57) **Abstract**

There are disclosed a compound of the formula (I): 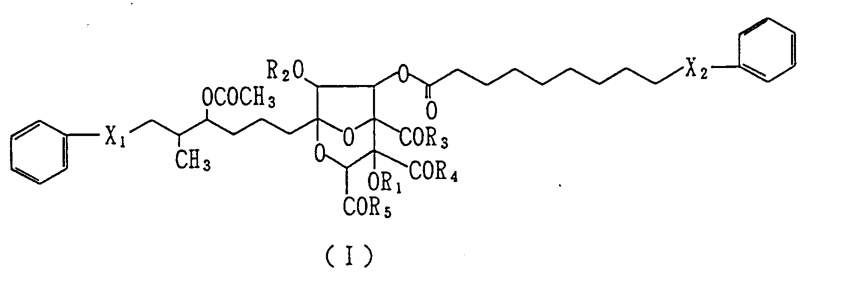
wherein each of X₁ and X₂ is ethylene or vinylene, each of R₁ and R₂ is hydrogen or an acyl group, and each of R₃, R₄ and R₅ is hydroxyl or an optionally substituted amino group, or a salt thereof; its production; and an antilipemic agent comprising it.

## Description

### FIELD OF THE INVENTION

The present invention relates to a novel compound TAN-1607A and its derivatives which are useful as an agent for treating hyperlipemia.

### BACKGROUND OF THE INVENTION

Hyperlipemia as well as hypertension and smoking are known to be three major risk factors of ischemic heart diseases. It is extremely important to control the cholesterol level in blood for prevention or treatment of ischemic heart disease, coronary arteriosclerotic disease and the like.

As medicaments to lower the cholesterol level in blood, various medicaments have been developed. Recently, agents for inhibiting cholesterol biosynthesis derived from microorganisms have been developed and marketed as medicaments such as Lovastatin (U.S. Patent No. 4,231,938), Simvastatin (U.S. Patent 4,444,784), Pravastatin (U.S. Patent 4,346,227) and the like each of which inhibits 3-hydroxy-3-methylglutaryl coenzyme A (HMG-CoA) reductase.

However, when HMG-CoA reductase is inhibited, biosynthesis of not only cholesterol but also other components required for living bodies such as ubiquinone, dolichol, heme A and the like is inhibited. Then, there is a fear of side effects caused by the inhibition.

It is known that these components are synthesized from farnesyl pyrophosphate which is present in the pathway of cholesterol biosynthesis. Therefore, in order to prevent side effects caused by the loss of them, it is preferable to inhibit enzyme systems after farnesyl pyrophosphate in the pathway of cholesterol biosynthesis. Examples of such an enzyme include squalene synthetases.

### OBJECTS OF THE INVENTION

The main object of the present invention is to provide novel compounds having squalene synthetase inhibitory activity.

Another object of the present invention is to provide processes for producing them.

Further another object of the present invention is to provide a novel antilipemic agent.

These objects as well as other objects and advantages of the present invention will become apparent to those skilled in the art from the following description.

### BRIEF EXPLANATION OF THE DRAWINGS

Fig. 1 shows the UV spectrum of TAN-1607A.

Fig. 2 shows the IR spectrum of TAN-1607A.

Fig. 3 shows the ¹³CNMR spectrum of TAN-1607A.

### SUMMARY OF THE INVENTION

Under these circumstances, the present inventors have studied from another viewpoint. As a result, it has been found that an active compound which strongly inhibits the biosynthesis of squalene is contained in culture medium of microorganisms belonging to a genus Cladosporium among a number of microorganisms separated from soil. Further, the present inventors have succeeded in isolating the active compound. Hereinafter, this compound is referred to as TAN-1607A. The compound is acidic fat-soluble substance containing carboxyl groups. It is analogous to the compounds for which patent applications have recently been filed as squalene synthetase inhibitors (EP-A-448,393, and U.S. Patent Nos. 5,026,554 and 5,053,425). The present inventors have examined its structure as well as its physical, chemical and biological properties in detail, and identified it as a novel compound completely different from the compounds in the above literatures. After further study, the present inventors have also been found that certain novel derivatives of TAN-1607A have similar activity to that of TAN-1607A. Thus, the present invention has been completed.

That is, according to the present invention, there is provided:
(1) A compound of the formula (I): 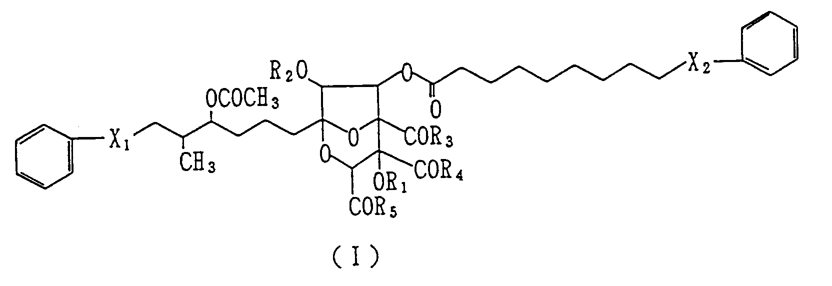 wherein each of X₁ and X₂ is ethylene or vinylene, each of R₁ and R₂ is hydrogen or an acyl group, and each of R₃, R₄ and R₅ is hydroxyl or an optionally substituted amino group, or a salt thereof;
(2) A process for producing a compound TAN-1607A of the formula (II): 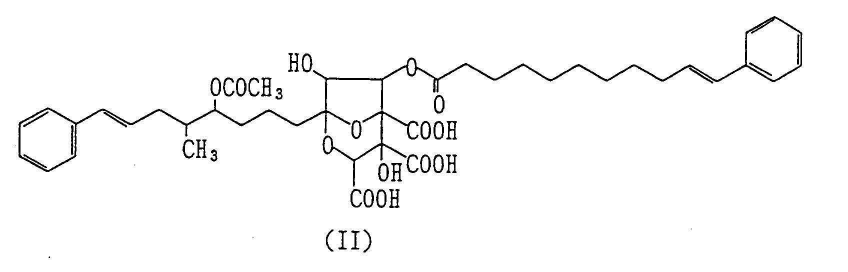 or a salt thereof, which comprises cultivating microorganisms belonging to a genus Cladosporium capable of producing the compound TAN-1607A in a medium to produce and accumulate the compound TAN-1607A in the culture and then collecting the compound TAN-1607A;
(3) A process for producing a compound of the formula (I): 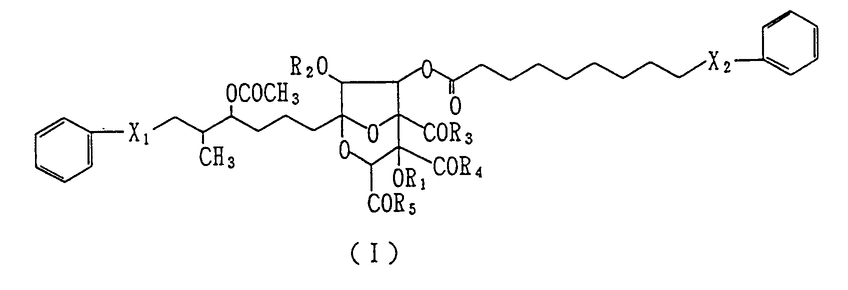 wherein each of X₁ and X₂ is ethylene or vinylene, each of R₁ and R₂ is hydrogen or an acyl group, and each of R₃, R₄ and R₅ is hydroxyl or an optionally substituted amino group, provided that, the case in which X₁ and X₂ are respectively vinylene, R₁ and R₂ are respectively hydrogen, and R₃, R₄ and R₅ are respectively hydroxyl is excluded, or a salt thereof, which comprises subjecting the compound TAN-1607A to acylation, amidation and/or catalytic hydrogenation; and
(4) An antilipemic agent comprising a compound of the formula (I): 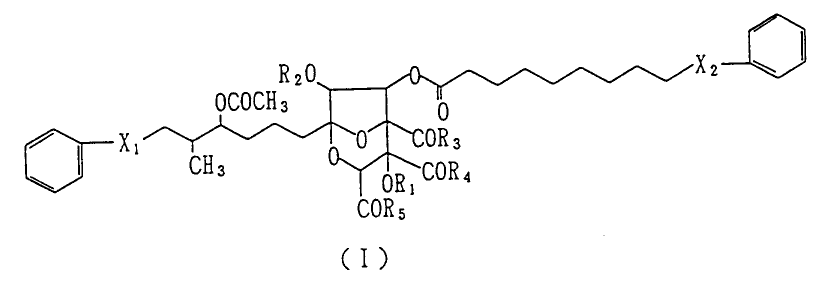 wherein each of X₁ and X₂ is ethylene or vinylene, each of R₁ and R₂ is hydrogen or an acyl group, and each of R₃, R₄ and R₅ is hydroxyl or an optionally substituted amino group, or a salt thereof.

### DETAILED DESCRIPTION OF THE INVENTION

The acyl group represented by R₁ or R₂ is preferably that derived from organic carboxylic acids such as formyl, alkanoyl (i.e. alkylcarbonyl), arylcarbonyl, aralkylcarbonyl, alkyloxycarbonyl, heterocyclic-carbonyl or the like. The above acyl group is preferably alkanoyl, arylcarbonyl and alkyloxycarbonyl. As examples of the alkyl in the alkanoyl (i.e. alkylcarbonyl) and alkyloxycarbonyl; the aryl in the arylcarbonyl; the aralkyl in the aralkylcarbonyl; and the heterocyclic group in the heterocyclic-carbonyl, there are such alkyl, aryl, aralkyl and heterocyclic group as described for the substituents of the optionally substituted amino group represented by R₃, R₄ or R₅ below.

The acyl group is more preferably acyl having 2 to 7 carbon atoms. As examples of the acyl group, there are acetyl, propionyl, butyryl, isobutyryl, valeryl, isovaleryl, pivaloyl, benzoyl, formyloxy, methoxycarbonyl, ethoxycarbonyl, propoxycarbonyl, isopropoxycarbonyl, butoxycarbonyl, tert-butoxycarbonyl, isobutoxycarbonyl and the like.

The above acyl group may be substituted with 1 to 3 appropriate substituents (e.g., hydroxyl, carboxyl, amino optionally substituted with C₁₋₆ alkyl, etc.).

As examples of the substituent in the optionally substituted amino group represented by R₃, R₄ or R₅, there are alkyl, cycloalkyl, aryl, aralkyl, acyl, heterocyclic groups and the like.

The above alkyl is preferably alkyl having 1 to 10 carbon atoms such as methyl, ethyl, propyl, isopropyl, butyl, isobutyl, sec-butyl, tert-butyl, pentyl, isopentyl, neopentyl, tert-pentyl, 1-ethylpropyl, hexyl, isohexyl, 1,1-dimethylbutyl, 2,2-dimethylbutyl, 3,3-dimethylbutyl, 2-ethylbutyl or the like. More preferably, they are alkyl having 1 to 6 carbon atoms such as methyl, ethyl, propyl, isopropyl, butyl, isobutyl, pentyl or the like.

The above cycloalkyl is preferably cycloalkyl having 3 to 8 carbon atoms such as cyclopropyl, cyclobutyl, cyclopentyl, cyclohexyl, cycloheptyl, cyclooctyl or the like. More preferably, it is cycloalkyl having 3 to 6 carbon atoms such as cyclopropyl, cyclobutyl, cyclopentyl, cyclohexyl or the like.

The above aryl is preferably aryl having 6 to 12 carbon atoms such as phenyl, naphthyl, biphenylyl or the like.

The above aralkyl is preferably aralkyl having 7 to 12 carbon atoms such as benzyl, phenethyl, benzhydryl, trityl, naphthylethyl or the like.

Examples of the acyl group include such an acyl group as described for R₁ or R₂ above.

As examples of the above heterocyclic group, there are 5- or 6-membered heterocyclic groups containing 1 to 4 heteroatoms selected from nitrogen atom, oxygen atom and sulfur atom. Examples of the heterocyclic group include pyrrolidino, 2-oxopyrrolidino, pyrrolidinyl, pyrrolyl, pyrazolyl, imidazolyl, furyl, thienyl, oxazolyl, isoxazolyl, isothiazolyl, thiazolyl, piperidino, piperidinyl, pyridyl, pyridazinyl, pyrazinyl, piperazinyl, pyrimidinyl, indolyl, 1,2,3-triazolyl, 1,2,4-triazolyl, 1,3,4-triazolyl, tetrazolyl, 1,3-dioxoranyl, morpholino, morpholinyl and the like. The heterocyclic group may form a condensed bicyclic group (e.g., 8-quinolyl, 8-purinyl, etc.) with a 5- or 6-membered ring (e.g., benzene, pyridine, cyclohexane, etc.).

The above alkyl as well as the above cycloalkyl, aryl, aralkyl, acyl, heterocyclic groups may further be substituted with 1 to 2 appropriate substituents (e.g., hydroxyl, carboxyl, amino optionally substituted with C₁₋₆ alkyl, etc.).

The substituent in the optionally substituted amino group is preferably an alkyl group or an aralkyl group, more preferably a C₁₋₆ alkyl group or a C₇₋₁₂ aralkyl group.

X₁ and X₂ are preferably vinylene.

The preferred compound of the formula (I) is that wherein X₁ and X₂ are respectively vinylene, R₁ and R₂ are respectively hydrogen, and each of R₃, R₄ and R₅ is hydroxyl or monoethylamino, preferably hydroxyl.

The more preferred compound of the formula (I) is that wherein X₁ and X₂ are respectively vinylene, R₁ and R₂ are respectively hydrogen, and R₃, R₄ and R₅ are respectively hydroxyl; X₁ and X₂ are respectively vinylene, R₁ and R₂ are respectively hydrogen, and R₃, R₄ and R₅ are respectively monoethylamino; X₁ and X₂ are respectively vinylene, R₁ is acetyl, R₂ is hydrogen, and R₃, R₄ and R₅ are respectively hydroxyl; X₁ and X₂ are respectively vinylene, R₁ and R₂ are respectively acetyl, and R₃, R₄ and R₅ are respectively hydroxyl; and X₁ and X₂ are respectively ethylene, R₁ and R₂ are respectively hydrogen, and R₃, R₄ and R₅ are respectively hydroxyl.

In the microbial production of the compound TAN-1607A of the present invention, any microorganism can be used so long as it belongs to the genus Cladosporium and is capable of producing the biologically active substance TAN-1607A. As the microorganism, there can be used, for example, Cladosporium cladosporioides FL-21431 strain separated from soil in India.

The bacteriological characteristics of FL-21431 strain are as follows:

### (1) Morphological characteristics

Aerial hyphae: Hyphae are colorless and have septa. The thickness (diameter) is 1.5 to 3.0 µm. The surface is smooth.

Conidiophores: It is not generally branched. It is 30 to 40 µm in height and 4 to 8 µm in width. The surface is smooth.

Conidia: The conidial chain branches dendritically from apexes of conidiophores or rami. Conidia form in a budding type. Four to eight conidia are in the site where the linkage is not branched. They are unicellular. The size is 1-3 x 1.5-4 µm. Conidia in various shapes such as egg-shape, lemon-shape, ellipsoidal shape or the like are observed. The surface is smooth and pale brown.

### (2) Growth in each medium:

### 1) Malt extract agar medium:

The growth is moderate. The diameter of the colony is 82 mm at 24°C on the 14th day. The surface is even and consists of thin mycelia. The outer edge is fringed regularly. Development of the aerial hyphae is slightly weak. Hyphae in the form of wool spread sparsely from the center to the intermediate region. The surface overall assumes pale yellow-white. The back assumes pale yellow-green from the center to the intermediate region and yellow-green in the outer region. Formation of soluble pigment is not observed.

### 2) Potato glucose agar medium

It grows in abundance. The diameter of the colony is 84 mm at 24°C on the 14th day. The surface is even and consists of mycelia which is slightly protuberant in the center. The outer edge is fringed somewhat irregularly. Development of the aerial hyphae is moderate. Hyphae in the form of wool spread radially and assume pale yellow-white to dark yellow-white. The reverse assumes dark yellow-brown spottedly in the center to the outer region and pale yellow-brown in the intermediate region. Formation of soluble pigment is not observed.

### 3) Czapek agar medium

It grows moderately. The diameter of the colony is 65 mm at 24°C on the 14th day. The surface consists of even mycelia. The outer edge is fringed regularly. Aerial hyphae are in the form of wool, spread overall and assume yellow-white. The reverse assumes dark red-brown in the center to the intermediate region and pale yellow-brown in the outer region. Formation of soluble pigment is not observed.

### 3) Oatmeal agar medium

It grows moderately. The diameter of the colony is 72 mm at 24°C on the 14th day. The surface is even and consists of mycelia which is slightly protuberant in the center to the intermediate region. The outer edge is fringed somewhat irregularly. Aerial hyphae in the form of wool are observed in the center to the intermediate region, and assume dark black-brown and yellow-white spottedly. In particular, numerous black conidia are observed in the center. The reverse assumes dark brown in the middle and dark brown to yellow-brown or pale yellow-brown in the intermediate region. Formation of soluble pigment is not observed.

Based on the above characteristics, by reference to "Separation, Cultivation and Characterization of mold (Kabi No Bunri, Baiyo To Dotei)" written by D. Malloch and translated by Shun-ichi Udagawa, 1983, Ishiyaku Shuppan Kabushiki Kaisha and "An illustrated Book of Fungi (Kinrui Zukan) (the last volume)" written by Shun-ichi Udagawa, Keisuke Tsubaki et al., 1983, Kodansha Scientific, it is obvious that this strain is identical with Cladosporium cladosporioides. The strain can be referred to as Cladosporium cladosporioides FL-21431.

The above Cladosporium cladosporioides FL-21431 strain has been deposited at Institute for Fermentation, Osaka (IFO), Japan under the accession number of IFO-32443 since April 21, 1992. It has also been deposited at Fermentation Research Institute, Agency of Industrial Science and Technology, Ministry of International Trade and Industry (FRI), Japan according to the Budapest Treaty under the accession number of FERM BP-3844 since April 27, 1992.

The microorganism which belongs to the genus Cladosporium can mutate naturally or with a mutagenic agent due to general nature of microorganisms. For example, many mutants can be obtained naturally or by radiation of rays such as X-rays, gamma rays, ultraviolet rays or the like; single spore isolation; treatment with various chemicals; cultivation in media containing chemicals; or other artificial means. Any such naturally or artificially obtainable mutants can be used in the process of the present invention so long as they have capability of producing TAN-1607A.

The media to be used in the cultivation in the present invention may be liquid or solid so long as they contain nutrients which the strain to be used can utilize. In the case of large-scale treatment, liquid media are suitably used. Assimilable carbon sources, nitrogen sources which can be digested, inorganic materials and micronutrients are appropriately formulated to the media.

As carbon sources, there can be used, for example, glucose, lactose, sucrose, maltose, dextrin, starch, glycerin, mannitol, sorbitol, fats and oils (e.g., soybean oil, olive oil, bran oil, sesame oil, lard oil, chicken oil, etc.) and the like. As nitrogen sources, there can be used, for exmple, meat extract, yeast extract, dried yeast, soybean flour, corn steep liquor, peptone, cotton seed flour, urea, ammonium salts (e.g., ammonium sulfate, ammonium acetate, etc.) and the like. Further, there can appropriately be used salts containing sodium, potassium, calcium, magnesium or the like; metal salts such as iron, manganese, zinc, cobalt, nickel or the like; salts of inorganic acids such as phosphoric acid, boric acid or the like; and salts of organic acids such as acetic acid, propionic acid or the like. In addition, the media may contain amino acids (e.g., glutamic acid, aspartic acid, alanine, lysine, valine, methionine, proline, etc.), peptides (e.g., dipeptides, tripeptides, etc.), vitamins (e.g., vitamin B₁, vitamin B₂, nicotinic acid, vitamin B₁₂, vitamin C, etc.), nucleic acids (e.g., purine, pyrimidine and derivatives thereof) or the like. Inorganic or organic acids, alkalis, buffers or the like may be added for pH adjustment of the media. Further, fats and oils, surfactants or the like may appropriately be added for foam control.

The cultivation can be carried out by means of shake culture, aeration and agitation culture or the like. For large-scale treatment, so called submerged culture is preferred.

Conditions of the cultivation varies depending upon the state of media, composition, kinds of strains and means for cultivation. Normally, the temperature is about 12°C to 44°C and the initial pH is about 5 to 9. In particular, the temperature is preferably about 20°C to 30°C in the middle period of the cultivation, and the initial pH is preferably about 6 to 8. The cultivation period varies depending upon the above conditions. The cultivation is preferably continued until the concentration of the biologically active compound becomes maximum. In the case of shake culture or aeration and agitation culture using liquid media, the time required for that purpose is normally about 1 to 10 days.

The method for collecting the desired compound TAN-1607A from the culture is illustrated below. Since the compound is fat-soluble in acidic conditions, general means utilizing this property can suitably be adopted.

Firstly, the medium is adjusted to pH of about 1.5 to 6, preferably about 2 to 4, and then an organic solvent immiscible with water such as dichloromethane, ethyl acetate, methyl isobutyl ketone, butanol or the like is added to extract TAN-1607A. The extract is washed with water and concentrated to give crude material containing TAN-1607A. Alternatively, the organic solvent layer can be extracted with a dilute aqueous alkaline solution to dissolve TAN-1607A in the aqueous layer. The aqueous layer dissolving TAN-1607A is adjusted to acidic pH again and extracted with the above organic solvent to dissolve TAN-1607A in the organic solvent layer.

In order to obtain pure TAN-1607A, various chromatography techniques can advantageously be used to purify the crude material. As the carrier, there can be used, for example, silica gel, crystalline cellulose, adsorptive resins [e.g., DIAION HP-20 (manufactured by Mitsubishi Chemical Industries, Japan), Sephadex LH-20 (manufactured by Pharmacia, Sweden)], anion exchange resins [e.g., Amberlite IRA-402 (manufactured by Amberlite, U.S.A.), Dowex-1 (manufactured by Dow and Chemical Co., U.S.A.)] or the like. These carriers are normally used in column chromatography. For elution of the active compound from the column, appropriate organic solvents can be used depending upon a kind of supports. Examples of such solvents include organic solvents such as dichloromethane, toluene, ethyl acetate, acetone, methanol alone or in combination thereof; mixed solvents of an organic solvent immiscible with water and an aqueous solution such as water, a dilute aqueous alkaline solution, a dilute aqueous acidic solution, an inorganic aqueous solution (e.g., saline, ammonium chloride solution, etc.), a buffer or the like. The eluted organic solvent containing the active substance is concentrated to give a powdered residue. Alternatively, when the mixed solvent containing an aqueous solution is used, the eluted mixed solvent is extracted with an appropriate organic solvent immiscible with water and concentrated to give a powderd residue.

In order to obtain the pure desired compound, the crude powder can be further purified by preparative high performance liquid chromatography (HPLC). As the carrier, octadecyl silane (ODS) type or silica gel type support can advantageously used. As the mobile phase, in the case of ODS for example, a mixed solution of methanol or acetonitrile and an aqueous solution containing salts can advantageously be used. The eluate containing the desired compound is extracted with an appropriate organic solvent immiscible with water. The extract is concentrated, and the residue is crystallized or powdered from the above appropriate organic solvent or the like to give the desired compound.

Physical and chemical properties of TAN-1607A obtained in Example 2 hereinafter are as follows:
(1) Appearance: Colorless powder
(2) Optical rotation: [α]_{D} +14° (c 0.12, methanol, 25°C)
(3) Molecular weight: m/z 817 (M+Na)⁺, 839 (M-H+2Na)⁺, 861 (M-2H+3Na)⁺, 883 (M-3H+4Na)⁺ (SI-Mass spectroscopy; NaOH was added)
(4) Elemental analysis (%):

| | | |
|---|---|---|
| Calcd. | C, 64.98; | H, 7.21 |
| Found | C, 64.97; | H, 6.85 |

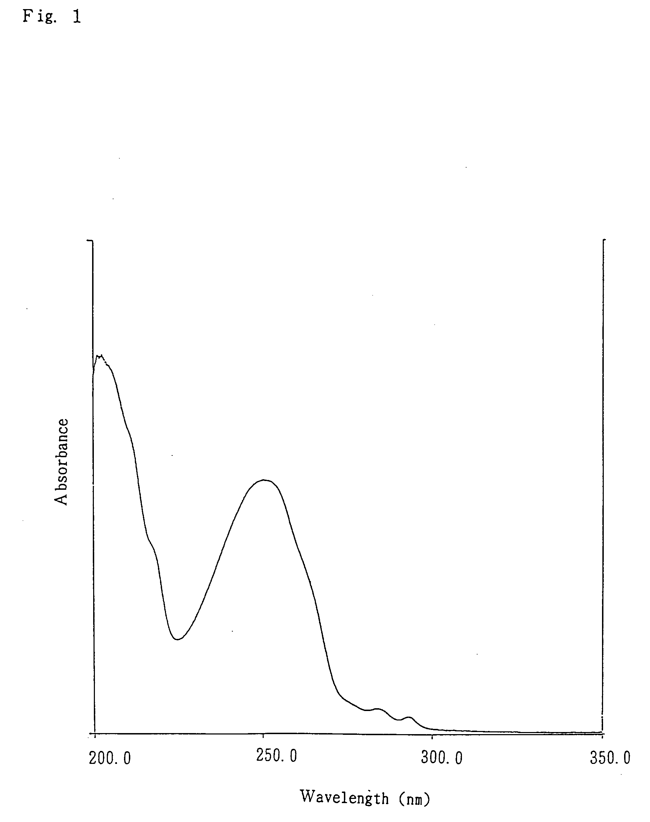

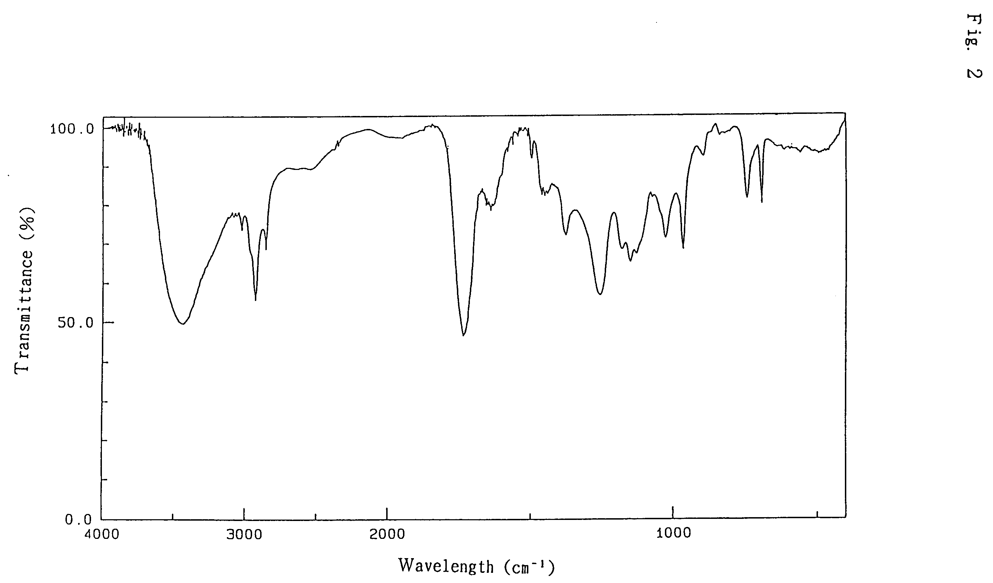

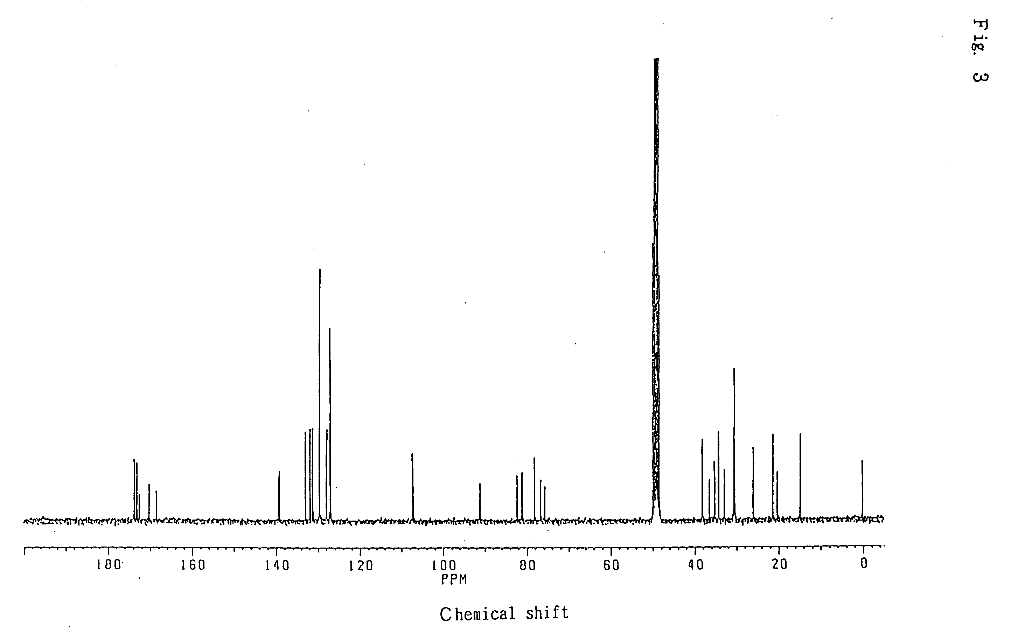

(5) Molecular formula: C₄₃H₅₄O₁₄
(6) Ultraviolet absorption (UV) spectrum: in methanol (Fig. 1) max. nm (ε) 248 (37,600), 283 (4,000), 292 (2,800)
(7) Infrared absorption (IR) spectrum: in KBr (Fig. 2), major absorptions are indicated (wavenumber, cm⁻¹) 3430, 2930, 1730, 1640, 1260, 1150, 1030, 960, 740, 690
(8) ¹³C nuclear magnetic resonance (NMR) spectrum: 75MHz, in CD₃OD (Fig. 3)
δ ppm 173.7 (Q), 173.1 (Q), 172.5 (Q), 170.2 (Q), 168.5 (Q), 139.3 (Q), 139.1 (Q), 132.9 (CH), 131.8 (CH), 131.2 (CH), 129.6 (CH), 129.5 x 4 (CH x 4), 128.0 (CH), 127.8 (CH), 127.0 x 2 (CH x 2), 126.9 x 2 (CH x 2), 107.2 (Q), 91.0 (Q), 82.2 (CH), 81.0 (CH), 78.0 (CH), 76.6 (CH), 75.6 (Q), 38.0 (CH₂), 38.0 (CH), 36.3 (CH₂), 35.0 (CH₂), 34.1 (CH₂), 32.7 (CH₂), 30.5 (CH₂), 30.4 (CH₂), 30.3 x 2 (CH₂ x 2), 30.1 (CH₂), 25.8 (CH₂), 21.2 (CH₃), 20.1 (CH₂), 14.7 (CH₃)
(9) Color reaction:
Positive: Phosphomolybdic acid, conc. Sulfuric acid reaction
Negative: Ninhydrin reaction, Barton reaction
(10) High performance liquid chromatography (HPLC):
Carrier: ODS, YMC-Pack A-312
Mobile phase: 46% acetonitrile/0.01M phosphate buffer (pH 6.3)
Flow rate: 2 ml/min
Detection: UV absorption, 214 nm and 254 nm
Elution time: 8.1 min
(11) Thin layer chromatography (TLC):
Carrier: Silica gel 60 F₂₅₄ (E. Merck AG.)
Developing solvent: chloroform:methanol:formic acid=80:20:4
Rf value: 0.25
(12) Property: acidic fat-soluble material
On the basis of the above physical and chemical data including the NMR spectrum, the structure of TAN-1607A has been established and is represented by the formula (II): 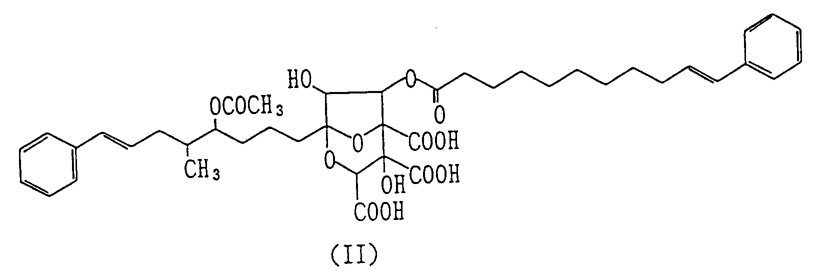 Since TAN-1607A is acidic material, treatment with an appropriate base gives a salt of TAN-1607A. The salt can be prepared by per se known methods.

Preferable salts of TAN-1607A are salts which are conventionally used in pharmaceutical fields. Examples of such salts include salts with inorganic bases such as alkaline metals (e.g., sodium, potassium, etc.), alkaline earth metals (e.g., calcium, magnesium, etc.) and ammonia; salts with organic bases such as methylamine, ethylamine, propylamine, isopropylamine, butylamine, tert-butylamine, dimethylamine, diethylamine, trimethylamine, triethylamine, pyridine, picoline, dicyclohexylamine, N,N'-dibenzylethylenediamine or the like; salts with amino acids such as lysine or the like.

The derivatives of TAN-1607A represented by the formula (I) can be prepared, for example, by subjecting the compound TAN-1607A to acylation, amidation and/or catalytic hydrogenation.

The acylation can be carried out by per se known methods, for example, by acylating the starting compound TAN-1607A or a salt thereof. As the acylating agent, there can be used, for example, organic carboxylic acids introducing the above acyl group represented by R₁ or R₂ or reactive derivatives thereof. Examples of such reactive derivatives of the carboxylic acids include acid halides, acid anhydrides, active amides, active esters, active thioesters or the like each of which can be prepared by conventional methods. Such reactive derivatives are illustrated as follows.
1) Acid halides:
   There can be used, for example, acid chlorides, acid bromides or the like.
2) Acid anhydrides:
   There can be used, for example, symmetric acid anhydrides, mono C₁₋₆ alkyl carbonic acid mixed anhydrides, mixed acid anhydrides composed of aliphatic carboxylic acids (e.g., acetic acid, pivalic acid, valeric acid, isovaleric acid, trichloroacetic acid, etc.), mixed acid anhydrides composed of aromatic carboxylic acids (e.g., benzoic acid, etc.) or the like. Examples of such symmetric acid anhydrides include C₁₋₆ alkylcarboxylic anhydides such as acetic anhydride, propionic anhydride, butanoic anhydride or the like.
3) Active amides:
   There can be used, for example, amides with pyrazole, imidazole, 4-substituted imidazole, dimethylpyrazole, benzotriazole or the like.
4) Active esters:
   There can be used, for example, methoxymethyl ester, 1-hydroxybenzotriazole ester, N-hydroxy-5-norbornene-2,3-dicarboximide ester, 4-nitrophenyl ester, 2,4-dinitrophenyl ester, trichlorophenyl ester, propargyl ester and pentachlorophenyl ester as well as esters with 1-hydroxy-1H-2-pyridone, N-hydroxysuccinimide, N-hydroxyphthalimide or the like.
5) Active thioesters:
   There can be used, for example, thioesters with heterocyclic group-thiols such as 2-pyridylthiol, 2-benzothiazolylthiol or the like.

The various reactive derivatives above can be selected depending upon a kind of a particular carboxylic acid.

Alternatively, as the acylating agent, there can also be used reactive derivatives of sulfonic acids which can introduce sulfonic acid acyl. Examples of such reactive derivatives of sulfonic acids include sulfonic acid halides such as methanesulfonyl chloride, benzylsulfonyl chloride, p-toluenesulfonyl chloride; symmetric acid anhydrides such as methanesulfonic anhydride, p-toluenesulfonic anhydride or the like; and the like.

The carboxylic acids, reactive derivatives thereof or reactive derivatives of sulfonic acids may be used in an amount of e.g. about 1 mol or more, preferably about 1 to 30 mol per 1 mol of the starting compound TAN-1607A.

This reaction can be carried out in a solvent which dose not hinder the reaction or in the absence of solvents. Examples of such a solvent include ketones (e.g., acetone, etc.), ethers (e.g., diethyl ether, tetrahydrofuran, dioxane, etc.), carboxylic acid (e.g., acetic acid, propionic acid, etc.), nitriles (e.g., acetonitrile, etc.), hydrocarbons (e.g., benzene, toluene, xylene, etc.), halogenated hydrocarbons (e.g., dichloromethane, chloroform, 1,2-dichloroethane, etc.), esters (e.g., ethyl acetate, etc.), amides (e.g., dimethylformamide, dimethylacetamide, etc.), tert-amines (e.g., triethylamine, tributylamine, N-methylmorpholine, N-methylpiperidine, N,N-dimethylaniline, etc.), pyridines (e.g., pyridine, picoline, lutidine, collidine, etc.) and the like. These solvents can be used alone or as mixed solvents thereof having an appropriate mixing ratio.

This acylation proceeds more advantageously by using a catalyst which can promote the acylation of the starting compound. As such a catalyst, for example, base catalysts or acid catalysts can be used. Examples of such base catalysts include tert-amines (e.g., aliphatic tert-amines (e.g., triethylamine, etc.), aromatic tert-amines (e.g., pyridine, α-, β- or γ-picoline, 2,6-lutidine, 4-dimethylaminopyridine, 4-(1-pyrrolidinyl)pyridine, dimethylaniline, diethylaniline, etc.), etc.), alkaline metal halide (e.g., potassium fluoride, anhydrous lithium iodide, etc.), organic acid salts (e.g., sodium acetate, etc) and the like. Examples of such acid catalysts include Lewis acids (e.g., anhydrous zinc chloride, anhydous aluminium chloride (AlCl₃), titanium tetrachloride (TiCl₄), tin tetrachloride (SnCl₄), antimony pentachloride, cobalt chloride, cupric chloride, boron trifluoride etherate, etc.), inorganic strong acids (e.g., sulfuric acid, perchloric acid, hydrogen chloride, hydrogen bromide, etc.), organic strong acids (e.g., benzenesulfonic acid, p-toluenesulfonic acid, trifluoroacetic acid, trichloroacetic acid, camphorsulfonic acid, etc.), acidic ion exchange resins (e.g., polystyrenesulfonic acid, etc.) and the like. Among the above catalysts, camphorsulfonic acid, pyridine, 4-dimethylaminopyridine or the like is preferable.

The amount of the catalyst to be used is a catalytic amount which can promote the acylation of the starting compound with carboxylic acids, and is normally about 0.001 to 10 mol, preferably about 0.001 to 1 mol per 1 mol of the starting compound (II).

The reaction temperature is not specifically limited and is normally about -30 to 100°C, preferably about 10 to 50°C. The reaction time is about several minutes to several tens hours (e.g., about 5 minutes to 30 hours, etc.).

The amidation can be carried out by per se known methods. For example, the compound TAN-1607A or a salt thereof is reacted with amines represented by the formula: 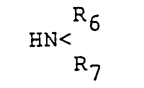
wherein R₆ and R₇ means hydrogen or the substituents in the optionally substituted amino represented by R₃, R₄ and R₅ as described above. The starting compound to be used in this reaction is preferably a reactive derivative having an activated carboxyl group. Examples of such a reactive derivative include the above acid halides, acid anhydrides, active esters, active thioesters and the like.

This reaction is sometimes carried out in the presence of a base. Examples of such a base include aliphatic tert-amines such as trimethylamine, triethylamine, tripropylamine, tri-n-butylamine and the like; tert-amines such as diisopropylethylamine, N-methylpiperidine, N-methylpyrrolidine, cyclohexyldimethylamine, N-methylmorpholine and the like; dialkylamines such as di-n-butylamine, diisobutylamine, dicyclohexylamine and the like; aromatic amines such as pyridine, lutidine, γ-collidine and the like; hydroxides or carbonates of alkaline metals (e.g., lithium, sodium, potassium, etc.) or alkaline earth metals (e.g., calcium, magnesium, etc.); and the like.

In this reaction, the amine is used normally in an amount of about 1 to 100 mol per 1 mol of the starting compound. Alternatively, the amine may be used in excess so long as it dose not hinder the reaction. When the base is used, the amount of the base to be used varies dependng upon a kind of the starting material (i.e., TAN-1607A or a salt thereof) and the reactive derivative of the caroboxylic acid compound as well as other reaction conditions, and it is normally about 1 equivalent to 30 equivalents, preferably about 1 equivalent to 10 equivalents based on the compound TAN-1607A.

This reaction is carried out normally in a solvent which dose not hinder the reaction. Examples of such a solvent include conventional organic solvents, for example, ethers such as dioxane, tetrahydrofuran, diethyl ether, diisopropyl ether or the like; esters such as ethyl acetate, ethyl formate or the like; halogenated hydrocarbons such as chloroform, dichloromethane, 1,2-dichloroethane, 1,1,1-trichloroethane or the like; hydrocarbons such as benzene, toluene, n-hexane or the like; amides such as N,N-dimethylformamide, N,N-dimethylacetamide or the like; nitriles such as acetonitrile or the like; and the like. These solvents can be used alone or in combination thereof. Among the above solvents, the above liquid bases can be used as a solvent. The reaction temperature is not specifically limited so long as the reaction can proceed, and is normally about -50°C to 150°C, preferably about -30°C to 50°C. The reaction time varies depending upon the starting material and base to be used, reaction temperature, and a kind of solvents. Normally, the reaction is completed within several tens minutes to several tens hours. In some instances, it takes several tens days.

The catalytic hydrogenation can be carried out by per se known methods. For example, it is carried out by shaking or stirring the compound TAN-1607A or a salt thereof in a solvent in the presence of a catalyst for catalytic hydrogenation in an atmosphere of hydrogen.

Examples of such a catalyst for catalytic hydrogenation include platinum catalysts such as platinum sponge, platinum asbestos, platinum black, platinum oxide, colloidal platinum or the like; palladium catalysts such as palladium sponge, palladium black, palladium oxide, palladium barium sulfate, palladium barium carbonate, palladium carbon, palladium silica gel, colloidal palladium or the like; nickel catalysts such as reduced nickel, nickel oxide, Raney nickel, Urushibara nickel or the like.

The hydrogenation can normally be carried out in a solvent. The solvent is not specifically limited so long as it dose not hinder the reaction. Examples of the solvent include water; alcohols such as methanol, ethanol, propanol, isopropanol or the like; ethers such as tetrahydrofuran, dimethoxyethane, dioxane or the like; amides such as dimethylformamide or the like; and mixed solvents thereof.

In this reaction, the catalyst is used in an amount conventionally based on the starting compound . The amount varies depending upon a kind of catalysts and other reaction conditions, and is about 0.01 to 500 % by weight preferably about 0.1 to 100 % by weight based on the starting compound.

The reaction temperature is not specifically limited, and is normally ice-cooling temperature to about 80°C, preferably in the vicinity of room temperature. The reaction time is about several minutes to several tens hours.

The desired compound (I) thus obtained can be isolated and purified by par se known methods such as concentration, liquid conversion, solvent conversion, solvent extraction, lyophilization, crystallization, recrystallization, fractionation, chromatography and the like.

The compound (I) can form a salt with an appropriate base. Examples of the salt include the same salts as those of TAN-1607A described above.

Since the desired compound (I) has two or more asymmetric carbon atoms in the fundamental skeleton, four or more stereoisomers exist theoretically. Each of these isomers and mixtures thereof are also included in the present invention. Further, different stereoisomers exist when its substituents have asymmetric carbon atoms. Each of these isomers and mixtures thereof are also included in the present invention.

The following Experiments illustrate biological activities of TAN-1607A. The determination of squalene synthetase inhibitory activity of TAN-1607A and the results are as follows.

### Experiment 1

### Preparation of an enzyme derived from rat liver

SD-Male rats (6 weeks old) were bled to death followed by removal of liver. The liver (c.a. 10 g) was washed with ice-cooled pysiological saline, homogenized in an ice-cooled buffer solution (15 ml) [100 mM potassium phosphate (pH 7.4), 15 mM nicotinamide, 2 mM MgCl₂] and centrifuged at 10,000 x g for 20 minutes (4°C). The resulting supernatant was further centrifuged at 105,000 x g for 90 minutes (4°C). Then, the precipitate was suspended in an ice-cooled 100 mM potassium phosphate buffer solution (pH 7.4) and centrifuged at 105,000 x g for 90 minutes (4°C) again. The precipitate (microsome fraction) thus obtained was suspended in an ice-cooled 100 mM potassium phosphate buffer solution (pH 7.4) so that the protein concentration became about 40 mg/ml (measured with BCA Protein Assay Kit (Pias)), and the suspension was used as the enzyme solution.

### Experiment 2

### Preparation of an enzyme derived from human cells

Cultivation was carried out on Dulbecco modified Eagle medium containing 10% fetal calf serum in the presence of 5% CO₂ at 37°C. The human hepatoma cells HepG2 (ca. 1 x 10⁹ cells) thus obtained were suspended in an ice-cooled buffer solution (10 ml) [100 mM potassium phosphate (pH 7.4), 30 mM nicotinamide, 2.5 mM MgCl₂], and the cells were crushed by ultrasonication (30 seconds, twice). From the resulting sonicate, a microsome fraction was obtained according to the same manner as that described in Experiment 1. This microsome fraction was suspended in an ice-cooled 100 mM potassium phosphate buffer solution (pH 7.4) so that the protein concentration became about 4 mg/ml, and this suspension was used as the enzyme solution.

### Experiment 3

### Determination of squalene synthetase inhibitory activity

Squalene synthetase inhibitory activity was determined as follows by using the enzyme solutions prepared in Experiments 1 and 2.

Each enzyme solution (protein content: 0.8 µg) prepared in Experiment 1 or 2 was added to a solution (total amount: 50 µl) containing 5µM [1-³H] farnesyl pyrophosphoric acid (specific activity: 25 µCi/µmol), 1 mM NADPH, 5 mM MgCl₂, 6 mM glutathion, 100 mM potassium phosphate buffer solution (pH 7.4) and a test compound (added as a solution in water or DMSO), and the mixture was allowed to react at 37°C for 45 minutes. A mixed solution (150 µl) of chloroform/methanol (1:2) was added to stop the reaction. Then, chloroform (50 µl) and 3N sodium hydroxide (50 µl) were added. The chloroform layer (under layer) (50 µl) containing the reaction products composed mainly of squalene and toluene-type liquid scintillator (3 ml) were mixed, and the radioactivity was measured with a liquid scintillation counter.

The results are shown in Table 1 in terms of the concentration which inhibits the radioactivity incorporated into the chloroform layer by 50% (IC₅₀, molarity (M)).

**Table 1**

| Squalene synthetase inhibitory activity of TAN-1607A | |
|---|---|
| Enzyme | IC₅₀ (M) |
| Rat enzyme | 1.5 x 10⁻⁹ |
| Human enzyme | 1.5 x 10⁻⁹ |

As is clear from Table 1, TAN-1607A inhibited the action of squalene synthetases derived from the human cells and rat liver at very low concentrations.

Further, no mice died in an acute toxicity test using mice in which the compound TAN-1607A (100 mg/kg) was administered intraperitoneally.

On the basis of the biological activity described above, the compound of the present invention is effective as an agent for lowering cholesterol level and useful as an agent for preventing and treating hyperlipemia in mammals including humans. Further, the compound may be provided as an agent for oral or parenteral administration having a dosage form suitable as a medicament. The agent can be prepared by mixing the compound with a pharmaceutically acceptable carrier. Examples of agents for parenteral administration include injections, agents for drip infusion, solutions, suspensions, suppositories and the like. Examples of agents for oral administration include capsules, tablets, syrups, powders, granules and the like.

Agents for parenteral administration such as injections may contain tonicity agents (e.g., glucose, sorbitol, mannitol, sodium chloride, etc.), preservatives (e.g., benzyl alcohol, chlorobutanol, methyl parahydroxybenzoate, etc.), anticoagulants (e.g., dextran sulfate, heparin, etc.), solution adjuvants (e.g., cyclodextrins, tweens, etc.), stabilizing agents (e.g., polyethylene glycol, polylactic acid, etc.) and the like. In administration, these compounds are dissolved in conventional aqueous diluents, and the resulting preparations are used as solutions. Examples of such diluents include an aqueous glucose solution, physiological saline, Ringer's solution, solutions for nutrition, and the like. Agents for oral administration may contain additives such as vehicles, binders, disintegrants, lubricants, colorants, corrigents, stabilizers and the like.

These preparations can be administered orally or parenterally. The dose varies depending upon a kind of subject diseases, severity of diseases, age of patients and the like. In the case of administration to human adult patients for treatment of diseases, the daily dose is normally about 0.2 mg to 50 mg, preferably about 0.5 to 30 mg per patient calculated as a content of the compound.

The following Examples further illustrates the present invention in detail but are not to be construed to limit the scope of the present invention. In the Examples, all the percents of medium compositions are percents (weight/volume), and all the ratios of solvents for elution in chromatography are ratios by volume unless otherwise indicated.

### Example 1

A seed culture medium composed of glucose (2%), maltose (3.0%), raw soybean flour (1.5%), corn steep liquor (1.0%), polypeptone (0.5%), yeast extract (0.3%) and sodium chloride (0.3%), pH 6.0, was dispecsed in 500 ml portions to 2 liter Sakaguchi flasks and sterilized. Cladosporium cladosporioides FL-21431 (5 platinum loops) previously grown on a slant medium composed of Bact Potato Dextrose Agar (manufactured by Difco, U.S.A.) was inoculated in the flasks, and incubated at 28°C for 2 days on a reciprocating shaker. The culture solution (1 liter) was transferred to a 200 liter fermentor containing a sterilized main culture (120 liter) medium composed of glucose (1.0%), dextrin (4.0%), raw soybean flour (0.5%), malt extract (0.5%), polypeptone (0.5%), yeast extract (0.2%), iron sulfate heptahydrate (0.05%), magnesium sulfate hyptahydhydrate (0.05%), manganese sulfate heptahydrate (0.05%), potassium phosphate (0.1%) and precipitated calcium carbonate (0.5%), pH 7.5. The main fermentation was incubated at 28°C under aeration (120 liters/min) and stirring (140 rpm) for 114 hours at an internal pressure of 1.0 kg/cm² to produce and accumulate TAN-1607A.

### Example 2

The culture solution (105 liters) obtained in Example 1 was adjusted to pH 3 followed by addition of ethyl acetate (100 liters), and the mixture was stirred for 30 minutes. Then the mixture was subjected to filtration by using Radiolite 600 (manufactured by Showa Kagaku Kogyo, Japan) as a filter aid, and the organic solvent layer and the aqueous layer were separated. The resulting organic solvent layer (85 liters) was washed with water (30 liters x 2) and concentrated to dryness. The residue was washed with hexane (1 liter x 2) to give an oil (92 g). The resulting oil was subjected to chromatography on silica gel (500 g, Kiesel gel 60, 70-230 mesh, manufactured by A. Merck, Germany) and eluted with chloroform/methanol/formic acid (90:10:0.2, 6 liters and 80:20:0.2, 2 liters) after washing with chloroform/methanol/formic acid (95:5:0.2, 6 liters). The eluate was concentrated to dryness to give an oil. The oil (3.8 g) was subjected to chromatography on silica gel (solvent: ethyl acetate/methanol/oxalic acid and ethyl acetate/methanol/formic acid) twice to give crude material (2.0 g). The crude material thus obtained was subjected to column chromatogrphy on Sephadex LH-20 (1 liter) and eluted with methanol.

The active fractions were collected and concentrated to dryness to give an oil (0.6 g). The oil was subjected to preparative HPLC (column: YMC-Pack S-363, I-15 (manufactured by Yamamura Kagaku Laboratory); mobile phase: 39% acetonitrile/0.01M phosphate buffer (pH 6.3)). The fractions found to contain TAN-1607A as a main component based on the HPLC analysis were collected and concentrated. The concentrate was adjusted to pH 3 and extracted with ethyl acetate. The extract was concentrated to dryness to give a residue (145 mg). The residue was subjected to preparative HPLC (column: YMC-Pack SH-343, S-15; mobile phase: 43% acetonitrile/0.01M phosphate buffer (pH 6.3)). The fractions indicating a single peak by the HPLC analysis were collected and concentrated. The concentrate was adjusted to pH 3 and extracted with ethyl acetate. The extract was washed with water and concentrated to dryness. The residue was treated with ether-hexane to give powder of TAN-1607A (62 mg).

### Example 3

### Preparation of the tetrahydro compound of TAN-1607A

TAN-1607A (0.22 g, purity: 86%) was dissolved in methanol (7.5 ml). The solution was stirred for 1.5 hours at room temperature in the presence of 10% palladium-carbon (22 mg) under an atmosphere of hydrogen. The reaction mixture was filtered through a membrane filter (0.5 µm, manufactured by Millipore) and concentrated to dryness to give an oil (213 mg). This oil was subjected to reversed phase preparative HPLC (column: ODS, YMC-Pack, SH-343; mobile phase: 45% acetonitrile/0.01M phosphate buffer, pH 6.3). The fractions of the elution volume of 530 to 800 ml were concentrated to about 30 ml. The concentrate was adjusted to pH 2.0, extracted with ethyl acetate (20 ml) twice, washed with water and saturated brine, dried over sodium sulfate and concentrated to dryness to give white powder of the tetrahydro compound (152 mg).

| Elemental analysis: | | | |
|---|---|---|---|
| Calcd. for C₄₃H₅₈O₁₄·1.5H₂O: | C,62.53; | H,7.44; | N,0.00 |
| Found: | C,62.26; | H,7.45; | N,0.00 |

¹³C-NMR (75 MHz, in CD₃OD, δ ppm): 173.7 (Q), 173.1 (Q), 172.5 (Q), 170.2 (Q), 168.5 (Q), 143.9 (Q), 143.7 (Q), 129.4 (CH), 129.4 (CH), 129.4 (CH), 129.4 (CH), 129.3 (CH), 129.3 (CH), 129.2 (CH), 129.2 (CH), 126.7 (CH), 126.6 (CH), 107.2 (Q), 91.0 (Q), 82.2 (CH), 81.0 (CH), 78.1 (CH), 76.6 (CH), 75.6 (Q), 37.3 (CH), 37.0 (CH₂), 36.9 (CH₂), 36.3 (CH₂), 35.0 (CH₂), 33.6 (CH₂), 32.7 (CH₂), 32.5 (CH₂), 30.7 (CH₂), 30.6 (CH₂), 30.5 (CH₂), 30.3 (CH₂), 30.3 (CH₂), 30.2 (CH₂), 30.1 (CH₂), 25.8 (CH₂), 21.1 (CH₃), 20.1 (CH₂), 14.8 (CH₃).

### Example 4

### Preparation of the 4-O-acetyl compound of TAN-1607A

TAN-1607A (376 mg, purity: 96%) was dissolved in acetic acid (4 ml) and acetic anhydride (2 ml), and the solution was stirred at room temperature for 17 hours. The reaction mixture was concentrated to dryness and subjected to reversed phase preparative HPLC (support: ODS, YMC-Pack, S-363; mobile phase: 42% acetonitrile/0.01M phosphate buffer, pH 6.3). The fractions of the elution volume of 720 to 810 ml were concentrated to about 20 ml. The concentrate was adjusted to pH 2.0, extracted with ethyl acetate (10 ml) twice, washed with water and saturated brine, dried over sodium sulfate and concentrated to dryness to give white powder of the 4-O-acetyl compound (129 mg).

| Elemental analysis: | | | |
|---|---|---|---|
| Calcd. for C₄₅H₅₆O₁₅·H₂O: | C,63.22; | H,6.84; | N,0.00 |
| Found: | C,63.20; | H,6.83; | N,0.14 |

¹³C-NMR (75MHz, in CD₃OD, δ ppm): 173.3 (Q), 172.9 (Q), 170.0 (Q), 169.6 (Q), 168.3 (Q), 168.2 (Q), 139.2 (Q), 139.0 (Q), 132.8 (CH), 131.7 (CH), 131.1 (CH), 129.6 (CH), 129.4 (CH), 129.4 (CH), 129.4 (CH), 129.4 (CH), 127.9 (CH), 127.8 (CH), 127.0 (CH), 127.0 (CH), 126.9 (CH), 126.9 (CH), 108.2 (Q), 89.1 (Q), 82.1 (Q), 81.8 (CH), 81.7 (CH), 78.0 (CH), 75.5 (CH), 38.1 (CH), 37.9 (CH₂), 35.5 (CH₂), 35.0 (CH₂), 34.0 (CH₂), 32.6 (CH₂), 30.5 (CH₂), 30.4 (CH₂), 30.3 (CH₂), 30.3 (CH₂), 30.1 (CH₂), 25.7 (CH₂), 21.3 (CH₃), 21.2 (CH₃), 20.2 (CH₂), 14.7 (CH₃).

### Example 5

### Preparation of the 4,7-di-O-acetyl compound of TAN-1607A

TAN-1607A (105 mg, purity: 86%) was dissolved in acetic acid (2 ml) and acetic anhydride (1 ml), and the solution was stirred at room temperature for 62 hours in the presence of 10-camphorsulfonic acid (2.6 mg). The reaction mixture was diluted with ether-hexane (3:1, 20 ml) and extracted with 2% aqueous sodium carbonate solution (12 ml) three times. The aqueous layers were collected, adjusted to pH 2.5 and extracted with ethyl acetate (20 ml) twice. The organic layer was washed with water and saturated brine, dried over sodium sulfate, concentrated to dryness to give crude powder (116 mg). The powder was subjected to reversed phase preparative HPLC (support: ODS, YMC-Pack, SH-343; mobile phase: 46% acetonitrile/0.01M phosphate buffer, pH 6.3). The fractions of the elution volume of 280 to 345 ml were concentrated to about 20 ml. The concentrate was adjusted to pH 2.0, extracted with ethyl acetae (10 ml) twice. The organic layer was washed with water and saturated brine, dried over sodium sulfate and concentrated to dryness to give white powder of the 4,7-di-O-acetyl compound (58 mg).

| Elemental analysis: | | | |
|---|---|---|---|
| Calcd. for C₄₇H₅₈O₁₆·1.5H₂O: | C,62.31; | H,6.79; | N,0.00 |
| Found: | C,62.10; | H,6.73; | N,0.11 |

¹³C-NMR (75MHz, CD₃OD, δ ppm): 173.0 (Q), 172.9 (Q), 171.0 (Q), 169.1 (Q), 168.1 (Q), 167.7 (Q), 139.3 (Q), 139.1 (Q), 132.9 (CH), 131.8 (CH), 131.1 (CH), 129.5 (CH), 129.5 (CH), 129.5 (CH), 129.5 (CH), 129.5 (CH), 127.9 (CH), 127.8 (CH), 127.0 (CH), 127.0 (CH), 126.9 (CH), 126.9 (CH), 107.4 (Q), 89.1 (Q), 81.7 (Q), 80.7 (CH), 78.8 (Q), 77.8 (CH), 75.5 (CH), 38.2 (CH), 37.9 (CH₂), 35.6 (CH₂), 34.9 (CH₂), 34.1 (CH₂), 32.5 (CH₂), 30.5 (CH₂), 30.4 (CH₂), 30.2 (CH₂), 30.2 (CH₂), 30.0 (CH₂), 25.7 (CH₂), 21.3 (CH₂), 21.1 (CH₃), 20.6 (CH₃), 20.1 (CH₂), 14.8 (CH₃).

### Example 6

### Preparation of the triethylamide of TAN-1607A

TAN-1607A (100 mg) was dissolved in dichloromethane (4 ml), and then 1-hydroxybenzotriazole (68 mg), ethylamine hydrochloride (42 mg), triethylamine (139 µl), 1-ethyl-3-(3-dimethylaminopropyl)-carbodiimide hydrochloride (97 mg) were added. The mixture was stirred at 0°C for 1 hour and then at 20°C for 15 hours. The reaction mixture was concentrated, and 2% aqueous sodium bicarbonate solution (50 ml) was added. The mixture was extracted with ethyl acetate (100 ml). The resulting organic solvent layer was washed with water, dried over sodium sulfate and concentrated to dryness to give white powder (112 mg). The powder was subjected to column chromatography on silica gel filled with chloroform, and eluted with chloroform/methanol (97:3) after washing with chloroform (60 ml) to obtain fractions (each 4 ml). The fractions 7 to 10 were collected and concentrated to dryness to give the triethylamide (48 mg) of TAN-1607A.

| Elemental analysis: | | | |
|---|---|---|---|
| Calcd. for C₄₉H₆₉N₃O₁₁: | C,66.68; | H,7.95; | N,4.78 |
| Found: | C,67.18; | H,7.94; | N,4.80 |

Infrared absorption (IR) spectrum (in KBr, major absorptions are indicated (wavenumber, cm⁻¹)): 3410, 2930, 1730, 1670, 1540, 1250, 960, 740.

¹³C-NMR (75MHz, in CDCl₃, δppm): 173.7 (Q), 173.0 (Q), 170.6 (Q), 168.8 (Q), 167.0 (Q), 139.2 (Q), 139.0 (Q), 133.0 (CH), 131.7 (CH), 131.2 (CH), 129.5 (CH), 129.5 (CH), 129.4 (CH), 129.4 (CH), 129.3 (CH), 128.0 (CH), 127.8 (CH), 127.0 (CH), 127.0 (CH), 126.9 (CH), 126.9 (CH), 107.4 (Q), 91.0 (Q), 82.4 (CH), 80.9 (CH), 77.6 (CH), 77.5 (CH), 74.8 (Q), 38.2 (CH), 38.1 (CH₂), 36.4 (CH₂), 35.2 (CH₂), 35.0 (CH₂), 34.9 (CH₂), 34.8 (CH₂), 34.1 (CH₂), 32.7 (CH₂), 30.5 (CH₂), 30.4 (CH₂), 30.3 (CH₂), 30.3 (CH₂), 30.1 (CH₂), 25.8 (CH₂), 21.2 (CH₃), 20.2 (CH₂), 15.2 (CH₃), 15.0 (CH₃), 14.6 (CH₃), 14.4 (CH₃).

## Claims

1. A compound of the formula (I): 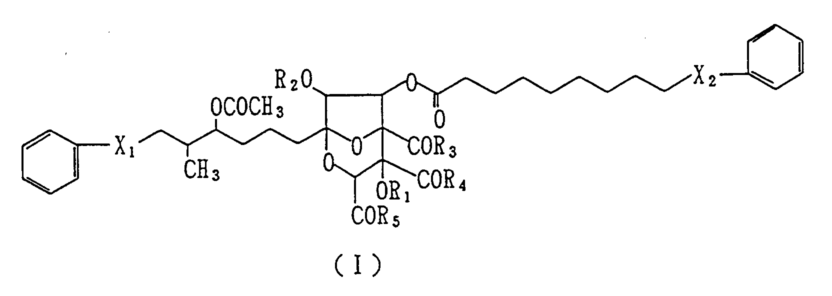 wherein each of X₁ and X₂ is ethylene or vinylene, each of R₁ and R₂ is hydrogen or an acyl group, and each of R₃, R₄ and R₅ is hydroxyl or an optionally substituted amino group, or a salt thereof.

2. A compound according to claim 1 wherein X₁ and X₂ are respectively vinylene.

3. A compound according to claim 1 wherein the acyl group is derived from an organic carboxylic acid.

4. A compound according to claim 1 wherein the acyl group is an acyl group having 2 to 7 carbon atoms.

5. A compound according to claim 1 wherein the optionally substituted amino group is an amino group which is mono- or di-substituted with an alkyl group.

6. A compound according to claim 5 wherein the alkyl group is an alkyl group having 1 to 6 carbon atoms.

7. A compound according to claim 1 wherein X₁ and X₂ are respectively vinylene, R₁ and R₂ are respectively hydrogen, and each of R₃, R₄ and R₅ is hydroxyl or monoethylamino.

8. A compound according to claim 7 wherein R₃, R₄ and R₅ are respectively hydroxyl.

9. A compound according to claim 7 wherein R₃, R₄ and R₅ are respectively monoethylamino.

10. A compound according to claim 1 wherein X₁ and X₂ are respectively vinylene, R₁ is acetyl, R₂ is hydrogen, and R₃, R₄ and R₅ are respectively hydroxyl.

11. A compound according to claim 1 wherein X₁ and X₂ are respectively vinylene, R₁ and R₂ are respectively acetyl, and R₃, R₄ and R₅ are respectively hydroxyl.

12. A compound according to claim 1 wherein X₁ and X₂ are respectively ethylene, R₁ and R₂ are respectively hydrogen, and R₃, R₄ and R₅ are respectively hydroxyl.

13. A process for producing a compound TAN-1607A of the formula (II): 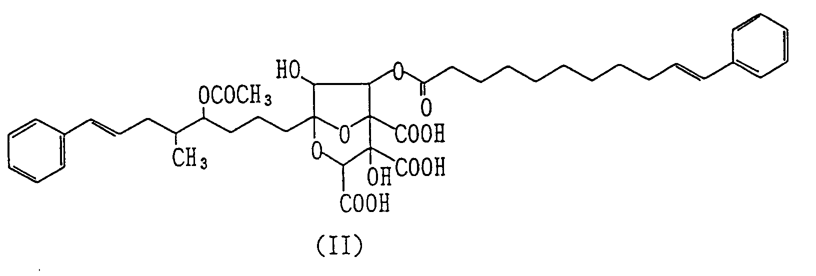 or a salt thereof, which comprises cultivating microorganisms belonging to a genus Cladosporium capable of producing the compound TAN-1607A in a medium to produce and accumulate the compound TAN-1607A in the culture, and then collecting the compound TAN-1607A.

14. A process for producing a compound of the formula (I): 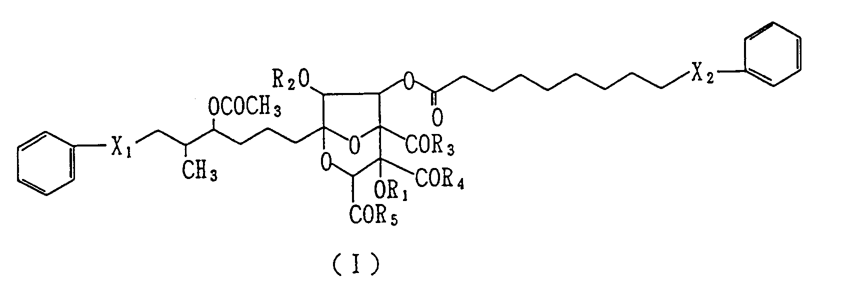 wherein each of X₁ and X₂ is ethylene or vinylene, each of R₁ and R₂ is hydrogen or an acyl group, and each of R₃, R₄ and R₅ is hydroxyl or an optionally substituted amino group, provided that, the case in which X₁ and X₂ are respectively vinylene, R₁ and R₂ are respectively hydrogen, and R₃, R₄ and R₅ are respectively hydroxyl is excluded, or a salt thereof, which comprises subjecting the compound TAN-1607A to acylation, amidation and/or catalytic hydrogenation.

15. An antilipemic agent comprising a compound of the formula (I): 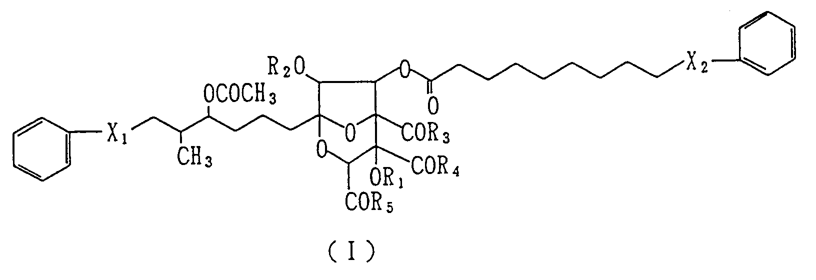 wherein each of X₁ and X₂ is ethylene or vinylene, each of R₁ and R₂ is hydrogen or an acyl group, and each of R₃, R₄ and R₅ is hydroxyl or an optionally substituted amino group, or a salt thereof.

16. An antilipemic agent according to claim 10 wherein X₁ and X₂ are respectively vinylene, R₁ and R₂ are respectively hydrogen, and R₃, R₄ and R₅ are respectively hydroxyl.

17. Use of a compound of the formula (I): 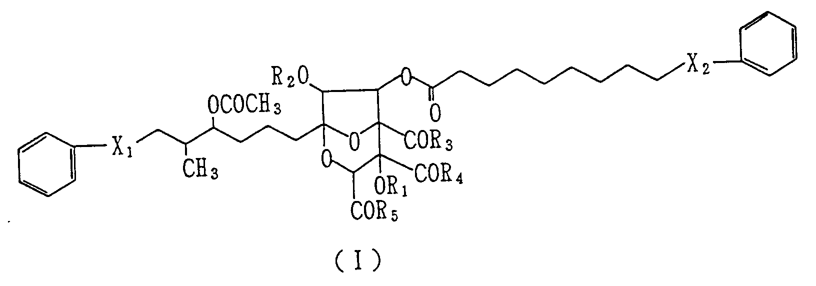 wherein each of X₁ and X₂ is ethylene or vinylene, each of R₁ and R₂ is hydrogen or an acyl group, and each of R₃, R₄ and R₅ is hydroxyl or an optionally substituted amino group, or a salt thereof in the manufacture of an antilipemic agent.

18. The use according to claim 17 wherein X₁ and X₂ are respectively vinylene, R₁ and R₂ are respectively hydrogen, and R₃, R₄ and R₅ are respectively hydroxyl.
